# EUROPEAN PATENT SPECIFICATION

(11) **EP 1 795 188 B1**
(45) Date of publication and mention of the grant of the patent: **31.03.2010**
(21) Application number: 06025475.2
(22) Date of filing: 08.12.2006
(51) Int. Cl.: A61K 9/70, A61K 9/06, A61K 9/00

(54) **Preparation for external use comprising an ionic liquid**
Zusammensetzung zur äusserlichen Anwendung enthaltend eine ionische Flüssigkeit
Composition à usage externe comprenant un liquide ionique

(30) Priority: 08.12.2005 JP 2005355107
(43) Date of publication of application: 13.06.2007
(73) Proprietor: NITTO DENKO CORPORATION, Osaka (JP)
(72) Inventor: Washiro, Satoko, Ibaraki-shi Osaka (JP); Hanatani, Akinori, Ibaraki-shi Osaka (JP)
(74) Representative: Grünecker, Kinkeldey, Stockmair & Schwanhäusser Anwaltssozietät

(56) References cited:
- EP-A- 0 189 861
- EP-A- 1 043 031
- EP-A1- 0 349 763
- EP-A1- 1 197 209
- EP-A1- 1 400 240
- US-A- 5 110 929
- US-A1- 2005 136 103

## Description

### Field of the Invention

This invention relates to an external preparation having an excellent percutaneous absorption ability for ionic drugs.

### Background of the Invention

Development of percutaneous absorption preparations has been advanced energetically, because of their ability to administer a drug for a prolonged period of time through the skin without accompanying pain and to prevent side effect. An ionic drug in the form of a salt is generally contained as the drug in such percutaneous absorption preparations, but since the keratin layer of the skin has a barrier function and also has high fat-soluble property, a drug in the form of a salt shows markedly low skin permeability in comparison with its free form. Thus, when an ionic drug in the form of a salt is used as the drug, it causes a problem in that the percutaneous absorption ability becomes insufficient.

Accordingly, with the aim of solving such a problem, for example in Patent Reference 1, an adhesive preparation for anti-inflammatory analgesic use has been proposed, in which the percutaneous absorption ability was increased by converting a non-steroidal anti-inflammatory analgesic agent in the form of a salt into its free form effected by adding thereto an organic acid having stronger acidity than that of the free form. However, such a pharmaceutical preparation causes problems in some cases, such as reduction of the stability of the drug due to conversion of the drug into its free form, and stimulation of the skin and reduction of the base material property due to the organic acid.

In addition, for the purpose of improving the percutaneous absorption ability, a method in which salts of ammonium compounds are blended with an anti-inflammatory agent in the form of a salt (Patent Reference 2), a method in which an addition salt compound of a basic substance is blended with a steroid anti-inflammatory agent in the form of a salt form (Patent Reference 3), a method in which a cationic surface active agent and an organic acid having 18 or less carbon atoms are blended with a percutaneous absorption drug (Patent Reference 4), a method in which an ionic compound having counter charge for an ionic medically active substance and capable of increasing hydrophobic property is blended with said substance (Patent Reference 5), a method in which an ionic drug is blended with its counterpart substance (Patent Reference 6), a method in which cromoglicic acid is blended with an ion pair compound (Non-patent Reference 1) and the like have been proposed. However, these methods are not always sufficient in terms of the percutaneous absorption ability of ionic drugs and have problems such as a possibility of lowering percutaneous absorption ability of the ionic drugs due to their crystallization when preserved for a prolonged period of time. Thus, it is hard to say that the percutaneous absorption ability of ionic drugs is sufficient in the conventional techniques, so that there is room for their improvement.
[Patent Reference 1] JP-B-07-47535
[Patent Reference 2] International Publication No. 2002/098396
[Patent Reference 3] International Publication No. 01/05381
[Patent Reference 4] JP-A-2003-321395
[Patent Reference 5] International Publication No. 99/33489
[Patent Reference 6] JP-A-2005-82512
[Non-patent Reference 1] Yakugaku Zasshi (Pharmacy Journal), 122 (9), 673 - 679 (2002)

### SUMMARY OF THE INVENTION

The invention has been made by taking such actual circumstances into consideration, and its problem to be solved is to provide an external preparation having excellent percutaneous absorption ability for ionic drugs.

With the aim of solving the aforementioned problem, the inventors have conducted intensive studies and found as a result that an ionic liquid can accelerate percutaneous absorption ability of ionic drugs, because it functions as a solubilizing agent for ionic drugs and thereby increases solubility of the ionic drugs.

That is, the invention is possessed of the following characteristics.
[1] An external preparation which comprises the following components (a) and (b);
   component (a): an ionic drug,
   component (b): an ionic liquid containing a cation and an anion.
[2] The external preparation described in the aforementioned [1], wherein the component (b) is a salt constitutedfromaquaternary nitrogen-containing cation, and at least one of an inorganic anion and an organic anion.
[3] The external preparation described in the aforementioned [2], wherein the quaternary nitrogen-containing cation is at least one selected from the group consisting of an imidazolium cation, a pyridinium cation, a pyrrolidinium cation and an ammonium cation.
[4] The external preparation described in the aforementioned [3], wherein the imidazolium cation is a cationic species represented by the following formula (1) : 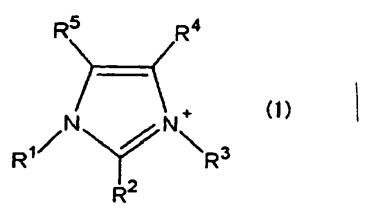 wherein R¹, R², R³, R⁴ and R⁵ each independently represents a hydrogen atom, a vinyl group, an alkyl or alkoxy group having from 1 to 25 carbon atoms, or an aryl or aralkyl group having from 6 to 25 carbon atoms.
[5] The external preparation described in any one of the aforementioned [1] to [4], wherein the component (a) is a drug having an anion residue.
[6] The external preparation described in any one of the aforementioned [1] to [5], wherein content ratio of the component (a) and component (b) (a:b) is from 1:0.5 to 1:10.
[7] The external preparation described in any one of the aforementioned [1] to [6], which is in the form of an adhesive preparation prepared by laminating an adhesive layer onto at least one side of a base material, wherein the adhesive layer comprises an adhesive containing the components (a) and (b).

According to the invention, an ionic liquid can accelerate percutaneous absorption ability of ionic drugs, because it functions as a solubilizing agent for ionic drugs and thereby increases solubility of the drugs in external preparations.

### Brief Description of the Drawings

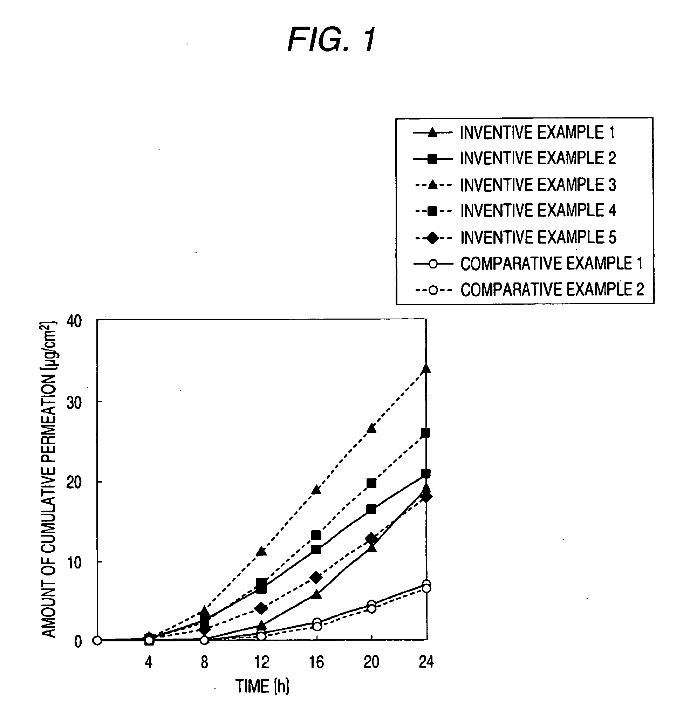

Fig. 1 is a graph showing cumulative amount of diclofenac permeated through the skin.

### DETAILED DESCRIPTION OF THE INVENTION

The following describes the invention in detail based on its preferable embodiments.

The external preparation of the invention is characterized in that it comprises an ionic drug as the component (a) and an ionic liquid as the component (b).

Firstly, the ionic drug as the component (a) is described.

The ionic drug functions as the active ingredient of the external preparation of the invention. In this case, the term "ionic drug" as used herein means a drug which has a cation residue or an anion residue. As the ionic drug, a drug having an anion residue is desirable. As the anion residue, for example, carboxylate (-COO⁻), sulfonate (-SO₃⁻) and phosphonate (-PO₄²⁻) can be cited. In addition, the ionic drug may be a pharmacologically acceptable salt, and examples of such a salt include sodium salt, potassium salt and the like alkali metal salts; magnesium salt, calcium salt and the like alkaline earth metal salts; aluminum salt and the like.

Illustrative examples of the ionic drug include salicylic acid, sulfanilamide, penicillin, analgesic anti-inflammatory agents (e.g., diclofenac sodium and sodium salicylate), antibiotics [e.g., β-lactam antibiotics (e.g., methicillin sodium, oxacillin sodium, cloxacillin sodium, ampicillin sodium, hetacillin sodium, carbenicillin sodium and the like penicillins; cefalotin sodium, cefazolin sodium, cefapirin sodium, cefatizin sodium, cefamezol sodium, cefroxy sodium and the like cephalosporins), oleandromycin phosphate, chloramphenicol succinate and fusidic acid sodium], chemotherapeutic agents (e.g., isoniazid sodium methanesulfonate), corticosteroid agents (e.g., hydrocortisone phosphate, dexamethasone sodium and prednisolone sodium phosphate) and the like.

In addition, according to the invention, when a pharmacologically acceptable salt is used as the ionic drug, it may be converted into its free form in the base material. For example, the salt can be converted into its free form in the base material, by allowing sodium hydroxide, potassium hydroxide, tetraethylamine, tetraethylammonium, ammonia, sodium caprylate or the like free form effecting agent to coexist in the base material together with the pharmacologically acceptable salt.

Amount of the component (a) to be contained is generally from 0.5 to 60% by weight, preferably from 1 to 30% by weight, based on the total weight of the external preparation. When such an amount is less than 0.5% by weight, it results in a tendency that the pharmacological effect cannot be obtained sufficiently, and when exceeds 60% by weight on the other hand, it results in a tendency that the pharmacological effect according to the increased amount cannot be obtained, which is economically disadvantageous.

Next, the ionic liquid as the component (b) is described.

The term "ionic liquid" as used herein means a salt containing a cation and an anion, having a melting point of from -90°C to 100°C and becomes a liquid state at a relatively low temperature in comparison with general inorganic salts having a melting point of about 800°C. In addition to its characteristics of non-volatility and low viscosity, such an ionic liquid has a characteristic property of having superior dissolving power for organic compounds and inorganic compounds due to its high polarity based on the aprotic ion structure.

Regarding the method for synthesizing the ionic liquid, an anion exchange method, an acid ester method, a neutralization method and the like can be employed.

As the cation which constitutes the ionic liquid, a quaternary nitrogen-containing cation, a phosphonium cation, a sulfonium cation and the like can be exemplified, of which a quaternary nitrogen-containing cation is preferable. Though the quaternary nitrogen-containing cation is not particularly limited, this is a general idea which also includes cyclic and aliphatic quaternary nitrogen-containing cations. As the quaternary nitrogen-containing cation, for example, an imidazolium cation, a pyridinium cation, a pyrrolidinium cation, a quaternary ammonium cation, pyrazolium cation and a triazolium cation can be cited, of which imidazolium cation, a pyridinium cation, a pyrrolidinium cation and a quaternary ammonium cation are preferable. As the ideal cation, the cation species represented by the following formula (1), (2), (3) or (4) can for example be cited, of which the imidazolium cation represented by the following formula (1) is more ideal from the viewpoint that a large number of various ionic liquids having low melting point can be prepared.

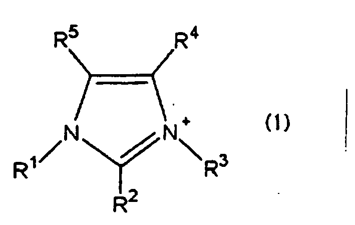

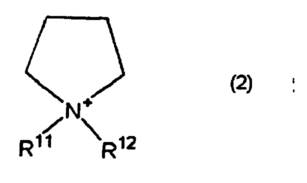

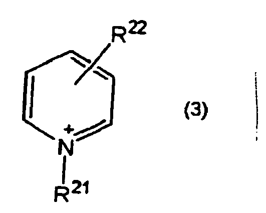

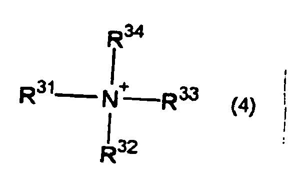

In the aforementioned formula (1), each of R¹, R², R³, R⁴ and R⁵ independently represents hydrogen atom, a vinyl group, an alkyl or alkoxy group having from 1 to 25 carbon atoms or an aryl or aralkyl group having from 6 to 25 carbon atoms. The aforementioned alkyl group and alkoxy group may be in the form of straight, branched or cyclic chain, of which straight chain is preferable. In addition, the aforementioned alkyl group, alkoxy group, aryl group and aralkyl group may be substituted with a halogen atom. As the alkyl group having from 1 to 25 carbon atoms, methyl group, ethyl group, n-propyl group, isopropyl group, n-butyl group, n-pentyl group, n-hexyl group, cyclohexyl group, decyl group, undecyl group, dodecyl group, tetradecyl group, hexadecyl group, octadecyl group, trifluoromethylgroup, pentafluoroethyl group, 2,2,2-trifluoroethyl group, heptafluoropropyl group and the like can be exemplified, and as the alkoxy group having from 1 to 25 carbon atoms, alkoxy groups formed through the binding of oxygen atom to the aforementioned alkyl groups (e.g., methoxy group) can be exemplified. As the aryl group having from 6 to 25 carbon atoms, phenyl group, tolyl group, xylyl group, biphenylyl group, naphthyl group, anthryl group, phenanthryl group, a phenyl group in which the p-position is substituted with fluorine atom or chlorine atom, a phenyl group in which the 3- and 4-positions are substituted with chlorine atoms, a phenyl group in which the m-position is substituted with trifluoromethyl group and the like can be exemplified. As the aralkyl group having from 6 to 25 carbon atoms, benzyl group, phenylethyl group, α-methylphenyl group, 1-methyl-1-phenylethyl group, a benzyl group in which the 3- and 4-positions of the aromatic ring are substituted with chlorine atoms and the like can be exemplified.

As the R¹, an alkyl group having from 1 to 10 carbon atoms is preferable, and an alkyl group having from 1 to 6 carbon atoms is more preferable. As the R³, an alkyl group having from 2 to 18 carbon atoms is preferable, and an alkyl group having from 2 to 12 carbon atoms is more preferable. As the R², R⁴ and R⁵, hydrogen atom or an alkyl group having from 1 to 6 carbon atoms is preferable, and hydrogen atom is more preferable.

In the aforementioned formula (2), each of R¹¹ and R¹² independently represents hydrogen atom, an alkyl or alkoxy group having from 1 to 25 carbon atoms or an aryl or aralkyl group having from 6 to 25 carbon atoms. The aforementioned alkyl group and alkoxy group may be in the form of straight, branched or cyclic chain, of which straight chain is preferable. In addition, the aforementioned alkyl group, alkoxy group, aryl group and aralkyl group may be substituted with a halogen atom. As the alkyl group, alkoxy group, aryl group and aralkyl group, the same groups described in the foregoing can be exemplified.

As the R¹¹, an alkyl group having from 1 to 4 carbon atoms is preferable, and an alkyl group having 1 or 2 carbon atoms is more preferable. As the R¹², an alkyl group having from 2 to 8 carbon atoms is preferable, and an alkyl group having from 2 to 4 carbon atoms is more preferable. In this connection, it is desirable that the R¹¹ and R¹² are asymmetric (different groups).

In the aforementioned formula (3), each of R²¹ and R²² independently represents hydrogen atom, an alkyl or alkoxy group having from 1 to 25 carbon atoms or an aryl or aralkyl group having from 6 to 25 carbon atoms. The aforementioned alkyl group and alkoxy group may be in the form of straight, branched or cyclic chain, of which straight chain is preferable. In addition, the aforementioned alkyl group, alkoxy group, aryl group and aralkyl group may be substituted with a halogen atom. As the alkyl group, alkoxy group, aryl group and aralkyl group, the same groups described in the foregoing can be exemplified.

As the R²¹, an alkyl group having from 2 to 12 carbon atoms is preferable, and an alkyl group having from 4 to 6 carbon atoms is more preferable. As the R²², hydrogen atom or an alkyl group having 1 or 2 carbon atoms is preferable, and hydrogen atom is more preferable.

In addition, in the aforementioned formula (4), each of R³¹, R³², R³³ and R³⁴ independently represents hydrogen atom, an alkyl or alkoxy group having from 1 to 25 carbon atoms or an aryl or aralkyl group having from 6 to 25 carbon atoms. The aforementioned alkyl group and alkoxy group may be in the form of straight, branched or cyclic chain, of which straight chain is preferable. In addition, the aforementioned alkyl group, alkoxy group, aryl group and aralkyl group may be substituted with a halogen atom. As the alkyl group, alkoxy group, aryl group and aralkyl group, the same groups described in the foregoing can be exemplified.

As the R³¹, R³², R³³ and R³⁴, hydrogen atom or an alkyl group having from 1 to 17 carbon atoms is preferable, and an alkyl group having from 1 to 8 carbon atoms is more preferable.

It is desirable that molecular structure of the cation is asymmetric. Since those which take asymmetric cation structure show a tendency of lowering melting point, drug dissolving property can be further improved. As such a cation, for example, those in which the R¹ and R³ in the aforementioned formula (1) are substituted with different substituent groups can be cited, and these are the same also in the aforementioned formulae (2) and (3). In this case, it is desirable that the R³ is an alkyl group having longer carbon chain than that of R³. By this, hydrophobic property is improved by the long chain alkyl group of R³, and high fluidity can be obtained due to reduced melting point depending on the combination of the alkyl groups of R¹ and R³, so that the functions as the solubilizing agent and percutaneous absorption accelerating agent of the ionic drug is improved. In this connection, when the carbon chain of R³ is too long, the molecular weight is increased accompanied thereby to cause a possibility of reducing thermodynamic activity of the ionic liquid and of increasing melting point, so that upper limit of the carbon chain is about 25. In addition, the cation existing in the ionic liquid may be a single species or two or more species.

In addition, as the anion which constitutes the ionic liquid, an anion capable of lowering melting point when used in combination with the aforementioned cation is suitably used. Such an anion may be either an inorganic anion or an organic anion, and is not particularly limited with the proviso that it is a pharmacologically acceptable substance. As the inorganic anion, a halide (e.g., chloride or bromide) ion, a fluorine-containing ion (e.g., tetrafluoroborate or hexafluorophosphate), nitrate and the like can be exemplified, and as the organic anion, methylsulfate, L-lactate, trifluorate/trifuryl ion [e.g., trifluoromethane sulfonate or bis(trifluoromethanesulfonyl) imide] and the like can be exemplified. As the anion, an inorganic anion is preferable, and a halide ion is particularly preferable.

When the balance of skin adhesiveness and cohesive strength is taken into consideration, amount of the component (b) to be contained is generally from 0.5 to 20% by weight, preferably from 1 to 15% by weight, based on the total weight of the external preparation. When such an amount is less than 0.5% by weight, it results in a tendency that the effects as a solubilizing agent and a percutaneous absorption accelerator cannot be exerted sufficiently, and when exceeds 20% by weight on the other hand, it results in a tendency that cohesive failure and blooming are apt to occur due to increase of adhesive strength.

In addition, containing ratio of the component (a) and component (b) (a:b) is generally from 1:0.5 to 1:10, preferably from 1:0.5 to 1:5, more preferably from 1:0.5 to 1:2. By this, percutaneous absorption can be further accelerated.

According to the invention, the external preparation maybe contained in a skin adhesive base. The skin adhesive base is not particularly limited, with the proviso that it can effect percutaneous absorption of an ionic drug as the active ingredient through the skin, by contacting with the skin. Illustratively, a base capable of constituting ointments, gels, emulsions, suspensions, cataplasmas, adhesive preparations (patches) and the like semisolid preparations or solid preparations; or lotions, liniments and the like solutions can be used.

As the ointment base, oils and fats, wax, hydrocarbon compounds and the like can be used generally as the hydrophobic bases. Illustratively, yellow petrolatum, white petrolatum, paraffin, liquid paraffin, Plastibase, silicone and the like mineral bases and yellow beeswax, animal and plant oils and fats and the like animal and plant bases can be exemplified. As the gel base, carboxyvinyl polymer, gel base, non-fat ointment, polyethylene glycol and the like as the hydrogel bases can be used. As the base for emulsions, hydrophilic ointment, vanishing cream and the like water/oil type bases; and hydrophilic petrolatum, purified lanolin, Aquaphor, Eucerin, Neoeserine, hydrous lanolin, cold cream, hydrophilic Plastibase and the like oil/water type bases can be exemplified. As the suspension base, lotions, FAPG (fatty alcohol-propylene glycol) base in which fine particles of stearyl alcohol, cetyl alcohol or the like are suspended in propylene glycol, namely lyogel base, and the like can be exemplified.

As the base for cataplasmas, gelatin, carboxymethylcellulose sodium, methyl cellulose, sodium polyacrylate, kaolin, polyvinyl alcohol, polyvinyl pyrrolidone, glycerol, propylene glycol, water and the like can be exemplified. The lotions are pharmaceutical preparations in which the active ingredient is finely and uniformly dispersed in an aqueous liquid and are divided into suspending lotions and emulsifying lotions. As the suspending agents, gum arabic, sodium alginate, carboxymethylcellulose sodium, methyl cellulose, bentonite and the like can be exemplified. As the emulsifying agents, sodium lauryl sulfate, sorbitan fatty acid ester and the like can be used. The liniments can be classified into an oily solution type, an alcohol solution type, an emulsion type and a suspension type. Water, ethanol, fatty oil, glycerol, soap, emulsifying agents, suspending agents, other additives and the like can be used in the liniments, and their examples include hard paraffin, soft paraffin, liquid paraffin, glycerol, paraffin oil, yellow beeswax, metallic soap, mucus (mucilage) ; natural oil [e.g., almond oil, corn oil, peanut oil, castor oil, olive oil, or a derivative thereof (e.g., Polyoxyl castor oil)]; and mutton tallow or a derivative thereof, afattyacidand/oranesterthereof (e.g., stearic acid, oleic acid, isopropyl myristate) and the like.

Also, it is desirable that the external preparation of the invention is stuck or applied to the region to be treated and then protected and fixed with a pressure sensitive adhesive sheet or a bandage, because this renders possible its use without staining clothing and fingers. In addition, it is desirable to use the external preparation of the invention in the form of an adhesive preparation prepared by forming it on one side of a sheet-like support, because amount of the drug to be administered can be easily controlled and the applying operation can also be carried out conveniently. In this connection, when the external preparation of the invention is used in the form of an adhesive preparation, it is suitable to form an adhesive layer on one side of the support using an adhesive as its main component, for the purpose of providing stickiness. Also, in order to prevent aimless adhesion of the adhesive layer to tools, containers and the like, or to prevent deterioration of the preparations, during their production, transportation or storage, it is desirable to cover and protect exposed face of the adhesive layer with a release liner until just before its application to the skin surface. Thereafter, the adhesive layer is exposed by releasing the release liner when used and stuck to the skin to effect the administration.

The adhesive to be contained in the adhesive layer is not particularly limited, with the proviso that it has stickiness at ordinary temperature (approximately from 20 to 30°C) and does not cause a rash and the like when contacted to the skin surface. It is desirable that the adhesive is constituted with an acryl system adhesive, a natural rubber system adhesive, a synthetic rubber system adhesive (synthetic isoprene rubber, polyisobutylene, styrene-butadiene rubber or styrene-isoprene-styrene rubber), a silicone system adhesive, a vinyl ester system adhesive, a vinyl ether system adhesive or the like, which is conventionally used in said technical field as an adhesive for medical use. Among these adhesives, it is particularly desirable to use an acryl system adhesive from the viewpoint of easy control of skin stimulation, skin adhesion and the like. Particularly, since lowering of glass transition temperature (Tg) can be expected by the addition of the ionic liquid, it is desirable to use an adhesive having high Tg for obtaining suitable adhesion characteristics, and, for example, an adhesive containing N-vinyl pyrrolidone, styrene or the like as its monomer unit is suitable.

The acryl system adhesive contains an acryl system polymer, and homopolymers of (meth)acrylic acid alkyl esters or copolymers thereof can be exemplified. It is desirable that the alkyl moiety of the (meth) acrylic acid alkyl ester is a straight chain or branched chain alkyl having from 4 to 12 carbon atoms. Illustrative examples of such a (meth)acrylic acid alkyl ester include (meth)acrylic acid butyl ester, (meth)acrylic acid t-butyl ester, (meth)acrylic acid pentyl ester, (meth) acrylic acid hexyl ester, (meth) acrylic acid heptyl ester, (meth) acrylic acid octyl ester, (meth) acrylic acid isooctyl ester, (meth)acrylic acid nonyl ester, (meth)acrylic acid isononyl ester, (meth)acrylic acid decyl ester, (meth)acrylic acid undecyl ester, (meth)acrylic acid dodecyl ester, (meth)acrylic acid 2-ethylhexyl ester and the like. Such a (meth)acrylic acid alkyl ester is contained in an amount of preferably 50% by weight or more, more preferably 60% by weight or more, based on the total weight of monomers constituting the acryl system polymer.

According to the invention, an additive agent other than the ionic liquid may be further contained in the adhesive layer, for the purpose of further increasing solubility of the ionic drug in the adhesive layer and also of rendering possible preservation of the ionic drug at a high concentration under a completely dissolved state. The additive agent may be any substance which has excellent compatibility with the adhesive, can sufficiently dissolve the ionic drug, does not cause periodical separation of adhesive component and additive agent and, moreover, does not exert a bad influence upon the adhesion characteristics and drug release property. As such an additive agent, for example, an ester of a fatty acid having from 12 to 16 carbon atoms, a monoglyceride of a fatty acid having from 8 to 10 carbon atoms, an ester of a dibasic acid having from 6 to 10 carbon atoms and the like higher fatty acid esters, and a polyoxyethylene alkyl ether having ethylene oxide added thereto in an average mol number of from 2 to 5, a polyoxyethylene alkyl phenyl ether having ethylene oxide added thereto in an average mol number of from 2 to 5 and the like nonionic surface active agents can be cited.

As the ester of a fatty acid having from 12 to 16 carbon atoms, esters obtained from hexyl laurate (C12), isopropyl myristate (C14), isopropyl palmitate (C16) and the like C₁₂ to C₁₆ fatty acids and C₁ to C₁₀ alkyl alcohols can be exemplified illustratively. As the monoglyceride of a fatty acid having from 8 to 10 carbon atoms, caprylic acid (C8) monoglyceride, capric acid (C10) monoglyceride and the like can be illustratively exemplified. As the ester of a dibasic acid having from 6 to 10 carbon atoms, diesters obtained from diisopropyl adipate (C6), dioctyl adipate, diethyl sebacate (C10) and the like C₆ to C₁₀ dibasic acids and C₁ to C₁₀ alkyl alcohols can be exemplified illustratively. The alkyl group in the polyoxyethylene alkyl ether having ethylene oxide added thereto in an average mol number of from 2 to 5 and polyoxyethylene alkyl phenyl ether having ethylene oxide added thereto in an average mol number of from 2 to 5 is possessed of from 6 to 18, preferably from 8 to 12 carbon atoms. As the polyoxyethylene alkyl ether, polyoxyethylene lauryl, polyoxyethylene oleyl ether, polyoxyethylene cetyl ether and the like can be illustratively exemplified. As the polyoxyethylene alkyl phenyl ether, polyoxyethylene nonyl phenyl ether, polyoxyethylene octyl phenyl ether and the like can be illustratively exemplified.

Among them, isopropyl myristate, caprylic acid monoglyceride, diisopropyl adipate and polyoxyethylene octyl phenyl ether having ethylene oxide added thereto in an average mol number of from 2 to 5 are desirable, of which isopropyl myristate is more desirable.

Also, according to the invention, an additive agent other than the aforementioned substances can be blended as occasion demands. As such an additive agent, for example, a stabilizing agent, an antiseptic or germicide, a suspending agent, a filler, a deterioration preventing agent, a tackifier, a plasticizer and the like can be used within such a range that they do not exert influence upon the pharmacological effect, in response to the main component of the skin adhering base, kind of the drug, or using method of the adhesive preparations and the like.

In addition, when the aforementioned additive agent is blended with an adhesive having a cross-linkable functional group, it is desirable to carry out the crosslinking treatment by an appropriate crosslinking means. By doing this, the adhesive becomes a so-called gelled state through the crosslinking treatment and can therefore prevent leaking of the additive agent component contained therein and provide the adhesive layer with an appropriate cohesive strength, even when the plasticization by the addition of the ionic liquid cannot be easily suppressed solely by the adhesive.

Aphysical crosslinking treatment by ultraviolet ray irradiation or electron beam irradiation or the like radiation exposure, a chemical crosslinking treatment which uses a crosslinking agent such as a polyisocyanate compound, an organic peroxide, an organic metal salt, a metal alcoholate, a metal chelate compound, a multifunctional compound or the like, and the like are employed in the crosslinking treatment.

Thickness of the adhesive layer is preferably from 20 to 100 µm, more preferably from 20 to 50 µm. By this, it can withstand its sticking to the skin surface for a prolonged period of time, and adhesive transfer to the skin surface at the time of its release and removal can be lessened.

Base material of the adhesive preparations is not particularly limited, with the proviso that it can support an adhesive layer which contains an ionic drug and an ionic liquid and is formed on its one side, but a material into which the ionic drug is not substantially transferred is generally used, and particularly desirable is a material having a flexibility of such an appropriate degree that it can follow a curve and a movement of the skin surface without generating a significant sense of incongruity when applied to the skin surface. Illustratively, a plastic film of a polyethylene system, polypropylene system, polyester system, polyvinyl acetate system, an ethylenevinyl acetate copolymer, a polyvinyl chloride system, a polyurethane system or the like, aluminum foil, tin foil or the like metal foil, a monolayer film consisting of non-woven fabric, woven fabric, paper or the like, a laminated film thereof and the like can be used.

Thickness of the base material is generally from 5 to 500 µm, preferably from 5 to 200 µm. In addition, in order to improve adhesiveness and anchor effect with the adhesive layer, it is desirable to apply a corona treatment, a plasma treatment, an oxidation treatment or the like to such a base material before laminating the adhesive layer.

The release liner is not particularly limited, with the proviso that it can be released easily from the adhesive layer when used, and, for example, a film of polyester, polyvinyl chloride, polyvinylidene chloride, polyethylene terephthalate or the like, wood free paper, glassine paper or the like paper, a laminated film of glassine paper or the like with a polyolefin film, and the like are used. It is desirable that a release treatment of such a release liner is carried out by applying a silicone resin, a fluoride resin or the like thereto before contacting with the adhesive layer. Thickness of the release liner is generally from 12 to 200 µm, preferably from 50 to 100 µm.

### Examples

The following illustratively describes the invention with reference to examples, but these examples do not restrict the invention.

### (Preparation of ionic liquid)

### (Preparation Example 1)

1-Bromododecane and 1.5 mol amounts of 1-methylimidazole were stirred at room temperature for 3 days in ethanol. Thereafter, this reaction solution was added dropwise to vigorously stirred diethyl ether, and the resulting precipitate was recovered and dried *in vacuo* to obtain an ionic liquid A represented by the following formula (5).

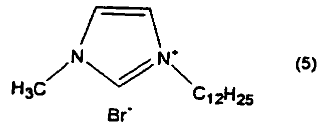

### (Preparation Example 2)

An ionic liquid B represented by the following formula (6) was obtained by the same method of Preparation Example 1, except that 1-bromohexane was used instead of 1-bromododecane.

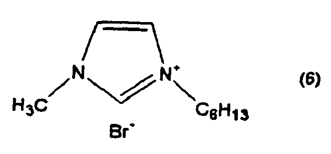

### (Preparation of external preparations)

### (Inventive Example 1)

Diclofenac sodium (50 mg) was weighed in a test tube equipped with a stopper, and then isopropyl myristate (IPM, 3000 mg) and the ionic liquid A (equimolar amount based on diclofenac sodium) were added thereto and vigorously stirred for 1 hour. Thereafter, centrifugation (500 rpm, 3 min) was carried out, and the supernatant fluid was recovered to obtain the product of interest.

### (Inventive Example 2)

Diclofenac sodium (50 mg) was weighed in a test tube equipped with a stopper, and then isopropyl myristate (IPM, 3000 mg) and the ionic liquid A (two molar amounts based on diclofenac sodium) were added thereto and vigorously stirred for 1 hour. Thereafter, centrifugation (500 rpm, 3 min) was carried out, and the supernatant fluid was recovered to obtain the product of interest.

### (Inventive Example 3)

The product of interest was obtained by the same method of Inventive Example 1, except that diclofenac was used instead of diclofenac sodium.

### (Inventive Example 4)

The product of interest was obtained by the same method of Inventive Example 2, except that diclofenac was used instead of diclofenac sodium.

### (Inventive Example 5)

The product of interest was obtained by the same method of Inventive Example 4, except that the ionic liquid B was used instead of the ionic liquid A.

### (Comparative Example 1)

Diclofenac sodium (50 mg) was weighed in a test tube equipped with a stopper, and then isopropyl myristate (IPM, 3000 mg) was added thereto and vigorously stirred for 1 hour. Thereafter, centrifugation (500 rpm, 3 min) was carried out, and the supernatant fluid was recovered to obtain the product of interest.

### (Comparative Example 2)

The product of interest was obtained by the same method of Comparative Example 1, except that diclofenac (50 mg) was used instead of diclofenac sodium.

Blending molar ratios of the ionic drugs and ionic liquids used in Inventive Examples 1 to 5 and Comparative Examples 1 and 2 are shown in Table 1.

**Table 1**

| | Diclofenac sodium | Diclofenac | Ionic liquid | |
|---|---|---|---|---|
| | | | A | B |
| Inventive Ex. 1 | 1 | - | 1 | - |
| Inventive Ex. 2 | 1 | - | 2 | - |
| Inventive Ex. 3 | - | 1 | 1 | - |
| Inventive Ex. 4 | - | 1 | 2 | - |
| Inventive Ex. 5 | - | 1 | - | 2 |
| Comparative Ex. 1 | 1 | - | - | - |
| Comparative Ex. 2 | - | 1 | - | - |

### (Drug solubility measurement on IPM)

Diclofenac concentration in the external preparations obtained in Inventive Examples 1 to 5 and Comparative Examples 1 and 2 was determined by high performance liquid chromatography (HPLC). The measuring conditions by HPLC are as follows. Results of the measurement are shown in Table 2, wherein the solubility in the table was shown as a solubility when the solubility (w/w %) of Comparative Example 1 was regarded as 1.
Column: L-column C8
Moving phase: mixed solution of 800 volumes of methanol, 200 volumes of purified water and 1 volume of phosphoric acid
Column temperature: 30°C
Flow rate: 1 ml/min
Detection method: measurement of absorbance at UV 280 nm

**Table 2**

| | Drug solubility in IPM |
|---|---|
| Inventive Example 1 | 0.6 |
| Inventive Example 2 | 1.7 |
| Inventive Example 3 | 13.1 |
| Inventive Example 4 | 17.9 |
| Inventive Example 5 | 9.8 |
| Comparative Example 1 | 1.0 |
| Comparative Example 2 | 8.7 |

### (Skin permeability test)

Cast-off skin (hydration with distilled water) of African rock python was applied to a cell for skin permeation test use (effective area 9 mm φ), and respective external preparations were added thereto from the upper side to carry out the skin permeation test for 24 hours. A degassed PBS(-) solution (phosphate buffered saline) was used as the receptor liquid and passed through it at a flow rate of about 10 ml/4 h. Sampling of the receptor liquid was carried out at predetermined intervals (4, 8, 12, 16, 20 and 24 hours), and concentration of the permeated diclofenac was determined by HPLC. Cumulative amount of diclofenac permeated through the skin when 4, 8, 12, 16, 20 and 24 hours passed after its administration (unit: µg/cm²) is shown in Fig. 1. The measuring conditions by HPLC are as follows.
Column: L-column C8
Moving phase: mixed solution of 800 volumes of methanol, 200 volumes of purified water and 1 volume of phosphoric acid
Column temperature: 30°C
Flow rate: 1 ml/min
Detection method: measurement of absorbance at UV 280 nm

In the case of Inventive Example 1, the drug solubility was a low value of 0.6 time in comparison with Comparative Example 1, but the permeability was improved 2 times or more. In the case of Inventive Example 2, the drug solubility became a higher value of 1.7 times and the permeability was also improved to approximately 3 times. Though reason for the improvement of permeability of Inventive Example 1 is not clear, it is considered that the ionic liquid having a highly hydrophobic alkyl chain exerted influence upon the skin and thereby induced an absorption accelerating action.

In the case of Inventive Examples 3 and 4, the drug solubility was improved to about 2 times in comparison with Comparative Example 2, and the permeability was also improved 3 times or more. When Inventive Examples 4 and 5 were compared, both of the drug solubility and permeability were improved in the case of Inventive Example 4. Based on this, it is considered that the lengthening of the alkyl chain of R³ of the imidazolium cation resulted in the improvement of permeability, because of the increased drug solubility despite of the lowered fluidity due to increased melting point of the ionic liquid itself.

## Claims

1. An external preparation which comprises the following components (a) and (b):
component (a): an ionic drug,
component (b): an ionic liquid containing a cation and an anion;
wherein the component (b) is a salt constituted from a quaternary nitrogen-containing cation, and at least one of an inorganic anion and an organic anion, and
wherein the quaternary nitrogen-containing cation is at least one selected from the group consisting of an imidazolium cation, a pyrrolidinium cation and a pyridinium cation represented by the following formulae (1) to (3): 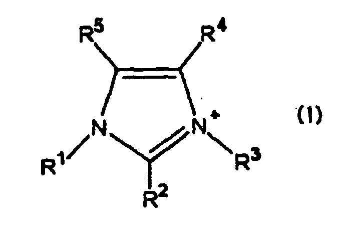 wherein each of R¹ and R³ independently represents a hydrogen atom, a vinyl group, an alkyl or alkoxy group having from 1 to 25 carbon atoms, or an aryl or aralkyl group having from 6 to 25 carbon atoms, wherein R¹ and R³ are substituted with different substituent groups, and wherein R², R⁴ and R⁵ independently represents a hydrogen atom, a vinyl group, an alkyl or alkoxy group having from 1 to 25 carbon atoms, or an aryl or aralkyl group having from 6 to 25 carbon atoms, 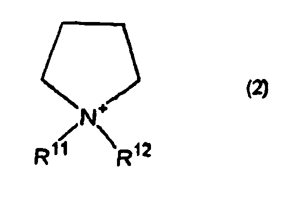 wherein R¹¹ and R¹² independently represents a hydrogen atom, an alkyl or alkoxy group having from 1 to 25 carbon atoms, or an aryl or aralkyl group having from 6 to 25 carbon atoms, wherein R¹¹ and R¹² are substituted with different substituent groups, 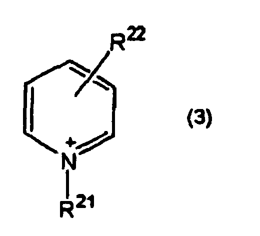 wherein R²¹ and R²² independently represents a hydrogen atom, an alkyl or alkoxy group having from 1 to 25 carbon atoms, or an aryl or aralkyl group having from 6 to 25 carbon atoms, wherein R²¹ and R²² are substituted with different substituent groups.

2. The external preparation of claim 1, wherein the component (a) is a drug having an anion residue.

3. The external preparation of claim 1 or 2, wherein the content ratio of the component (a) and the component (b) (a:b) is from 1:0.5 to 1:10.

4. The external preparation of any of claims 1 to 3, which is in the form of an adhesive preparation where an adhesive layer is laminated onto at least one side of a base material, wherein the adhesive layer comprises an adhesive containing the components (a) and (b).

## Patentansprüche

1. Äußerliche Zubereitung, welche die folgenden Komponenten (a) und (b) umfasst:
Komponente (a): ein ionisches Arzneimittel,
Komponente (b): eine ionische Flüssigkeit enthaltend ein Kation und ein Anion;
wobei die Komponente (b) ein Salz ist, das aus einem quartären Stickstoff enthaltenden Kation und wenigstens einem von einem anorganischen Anion und einem organischen Anion gebildet ist, und wobei das quartären Stickstoff enthaltende Kation wenigstens eines ist, das ausgewählt ist aus der Gruppe bestehend aus einem Imidazoliumkation, einem Pyrrolidiniumkation und einem Pyridiniumkation, die durch die folgenden Formeln (1) bis (3) wiedergegeben sind: 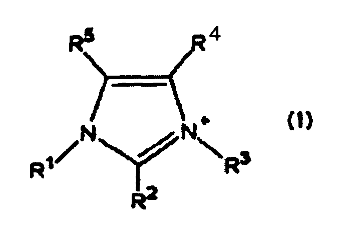 wobei jedes von R¹ und R³ unabhängig voneinander ein Wasserstoffatom, eine Vinylgruppe, eine Alkyl- oder Alkoxygruppe mit 1 bis 25 Kohlenstoffatomen oder eine Aryl- oder Aralkylgruppe mit 6 bis 25 Kohlenstoffatomen bedeutet, wobei R¹ und R³ mit verschiedenen Substituentengruppen substituiert sind, und wobei R², R⁴ und R⁵ unabhängig voneinander ein Wasserstoffatom, eine Vinylgruppe, eine Alkyl- oder Alkoxygruppe mit 1 bis 25 Kohlenstoffatomen oder eine Aryl- oder Aralkylgruppe mit 6 bis 25 Kohlenstoffatomen bedeuten, 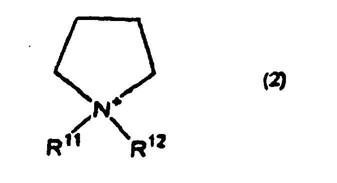 wobei R¹¹ und R¹² unabhängig voneinander ein Wasserstoffatom, eine Alkyl- oder Alkoxygruppe mit 1 bis 25 Kohlenstoffatomen oder eine Aryl- oder Aralkylgruppe mit 6 bis 25 Kohlenstoffatomen bedeuten, wobei R¹¹ und R¹² mit verschiedenen Substituentengruppen substituiert sind, 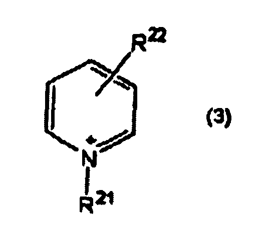 wobei R²¹ und R²² unabhängig voneinander ein Wasserstoffatom, eine Alkyl- oder Alkoxygruppe mit 1 bis 25 Kohlenstoffatomen oder eine Aryl- oder Aralkylgruppe mit 6 bis 25 Kohlenstoffatomen bedeuten, wobei R²¹ und R²² mit verschiedenen Substituentengruppen substituiert sind.

2. Äußerliche Zubereitung nach Anspruch 1, wobei die Komponente (a) ein Arzneimittel mit einem Anionrest ist.

3. Äußerliche Zubereitung nach Anspruch 1 oder 2, wobei das Verhältnis der Gehalte der Komponente (a) und der Komponente (b) (a:b) 1:0,5 bis 1:10 beträgt.

4. Äußerliche Zubereitung nach einem der Ansprüche 1 bis 3, welche in Form einer klebenden Zubereitung vorliegt, wobei eine Klebschicht auf wenigstens eine Seite eines Grundmaterials laminiert ist, wobei die Klebschicht einen Klebstoff umfasst, der die Komponenten (a) und (b) enthält.

## Revendications

1. Préparation externe qui comprend les constituants (a) et (b) suivants :
constituant (a) : un médicament ionique,
constituant (b) : un liquide ionique contenant un cation et un anion ;
dans laquelle le constituant (b) est un sel constitué d'un cation contenant de l'azote quaternaire et d'au moins un parmi un anion inorganique et un anion organique, et dans laquelle le cation contenant de l'azote quaternaire est au moins un cation choisi dans le groupe constitué d'un cation imidazolium, d'un cation pyrrolidinium et d'un cation pyridinium, représenté par les formules (1) à (3) suivantes : 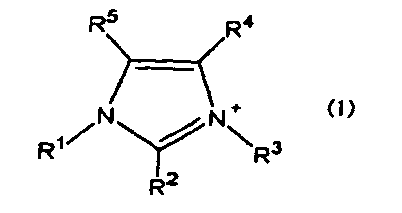
dans laquelle chacun de R¹ et R³ représente indépendamment un atome d'hydrogène, un groupe vinyle, un groupe alkyle ou alcoxy ayant de 1 à 25 atomes de carbone ou un groupe aryle ou aralkyle ayant de 6 à 25 atomes de carbone, où R¹ et R³ sont substitués avec des groupes substituants différents, et où R², R⁴ et R⁵ représentent indépendamment un atome d'hydrogène, un groupe vinyle, un groupe alkyle ou alcoxy ayant de 1 à 25 atomes de carbone, ou un groupe aryle ou aralkyle ayant de 6 à 25 atomes de carbone, 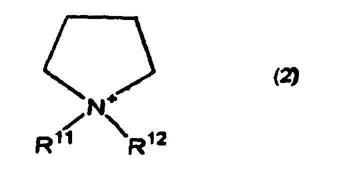 dans laquelle R¹¹ et R¹² représentent indépendamment un atome d'hydrogène, un groupe alkyle ou alcoxy ayant de 1 à 25 atomes de carbone, ou un groupe aryle ou aralkyle ayant de 6 à 25 atomes de carbone, où R¹¹ et R¹² sont substitués avec des groupes substituants différents, 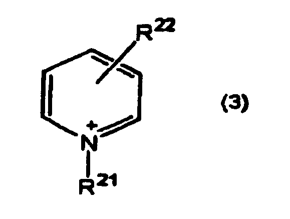 dans laquelle R²¹ et R²² représentent indépendamment un atome d'hydrogène, un groupe alkyle ou alcoxy ayant de 1 à 25 atomes de carbone, ou un groupe aryle ou aralkyle ayant de 6 à 25 atomes de carbone, dans laquelle R²¹ et R²² sont substitués avec des groupes substituants différents.

2. Préparation externe selon la revendication 1, dans laquelle le constituant (a) est un médicament présentant un résidu d'anion.

3. Préparation externe selon la revendication 1 ou 2, dans laquelle le rapport de teneur du constituant (a) et du constituant (b) (a:b) est de 1:0,5 à 1:10.

4. Préparation externe selon l'une quelconque des revendications 1 à 3, laquelle est dans la forme d'une préparation adhésive où une couche adhésive est stratifiée sur au moins un côté d'un matériau de base, dans laquelle la couche adhésive comprend un adhésif contenant les constituants (a) et (b).
